# EUROPÄISCHE PATENTSCHRIFT

(11) **EP 0 764 634 B1**
(45) Veröffentlichungstag und Bekanntmachung des Hinweises auf die Patenterteilung: **14.06.2000**
(21) Anmeldenummer: 96113617.3
(22) Anmeldetag: 26.08.1996
(51) Int. Cl.: C07C 271/14, C07C 271/46

(54) **N-(4-Brom-2-fluorbenzyl)carbamate sowie ein Verfahren zu ihrer Herstellung**
N-(4-bromo-2-fluorobenzyl)carbamates and process for their preparation
N-(4-bromo-2-fluorobenzyl)carbamates et procédé de leur préparation

(30) Priorität: 21.09.1995 DE 19535083
(43) Veröffentlichungstag der Anmeldung: 26.03.1997
(73) Patentinhaber: Clariant GmbH, 65929 Frankfurt am Main (DE)
(72) Erfinder: Beye, Norbert, Dr., 65779 Kelkheim (DE); Mack, Karl-Ernst, Dr., 65207 Wiesbaden (DE)

(56) Entgegenhaltungen:
- WO-A-94/10132
- US-A- 5 194 661
- CHEMICAL ABSTRACTS, vol. 72, no. 25, 22.Juni 1970 Columbus, Ohio, US; abstract no. 132344m, XP002067563 & JP 45 003 770 A (MITSUI TOATSU CHEMICALS CO., LTD.) 7.Februar 1970

## Beschreibung

Die Erfindung betrifft N-(4-Brom-2-fluorbenzyl)carbamate sowie ein Verfahren Zu ihrer Herstellung.

Die erfindungsgemäßen N-(4-Brom-2-fluorbenzyl)carbamate sind Ausgangsprodukte für stickstoffhaltige Heterocyclen, Pharmazwischenstufen und Pflanzenschutzmittel. Derartige Verbindungen sind insbesondere bei der Synthese neuer Aldose-Reduktase-Inhibitoren von Bedeutung. (K. Ogawva, I. Yamawaki, Y. I. Matsusita, N. Nomura, P. F. Kador, J. H. Kinoshita, Eur. J. Med. Chem. 28, 769, (1993)).
In der Patentschrift WO 94 10 132 A1 werden substituierte Benzylcarbamate mit herbiziden Eigenschaften beschrieben. Über die Synthese von N-Benzylcarbamaten wird in JP 70 03 770 [C.A. 72: 132344m (1970)] berichtet.

Es bestand daher ein Bedarf, neue Verbindungen dieser Substanzklasse bereitzustellen, um einerseits die Synthesemöglichkeiten bisher bekannter Produkte zu erweitern und andererseits neue Produkte zugänglich zu machen.

Diese Aufgabe wird gelöst durch N-(4-Brom-2-fluorbenzyl)carbamate der allgemeinen Formel (I), 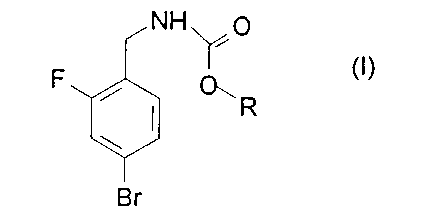 wobei R für (C₁-C₁₂)Alkyl, Benzyl oder Phenyl steht, wobei der Benzyl- und Phenylrest auch durch (C₁-C₃)Alkyl, (C₁-C₃)Alkoxy substituiert sein können.

Besonders interessant sind Verbindungen der Formel (I), worin R für (C₁-C₄)Alkyl, Benzyl oder Methoxybenzyl steht.

Wichtige Verbindungen sind O-Benzyl-N-(4-brom-2-fluorbenzyl)carbamat, O-Methyl-N-(4-brom-2-fluorbenzyl)carbamat, O-Ethyl-N-(4-brom-2-fluorbenzyl)carbamat, O-p-Methoxybenzyl-N-(4-brom-2-fluorbenzyl)carbamat, O-Butyl-N-(4-brom-2-fluorbenzyl)carbamat.

Die Erfindung betrifft ferner ein Verfahren zur Herstellung von Verbindungen der Formel (I), dadurch gekennzeichnet, daß ein 4-Brom-2-fluorbenzylhalogenid mit einem Alkali- oder Ammoniumcyanat in einem dipolar aprotischen Lösungsmittel in Gegenwart des Alkohols ROH umgesetzt wird.

Hierbei hat es sich bewährt, 4-Brom-2-fluorbenzylbromid, Kaliumcyanat und als Lösungsmittel DMF einzusetzen und bei einer Temperatur von 30 bis 150°C, insbesondere 80 bis 140°C, bevorzugt 100 bis 120°C, zu arbeiten.

Die Reaktion läuft im allgemeinen innerhalb von 1 bis 3 h ab. Es hat sich als günstig erwiesen, das Cyanat und den Alkohol im Überschuß einzusetzen, insbesondere hat sich eine Einsatzmenge von 1,1 bis 3 mol Cyanat und 1,1 bis 4 mol Alkohol pro Mol Benzylhalogenid bewährt.

Die nachfolgenden Beispiele dienen zur Erläuterung der Erfindung, ohne sie darauf zu beschränken.

### Beispiel 1

### Synthese von O-Benzyl-N-(4-brom-2-fluorbenzyl)carbamat

In einem 500 ml 2-Hals-Rundkolben mit Rückflußkühler und Trockenrohr werden trockenes und aminfreies DMF (180 ml) und Benzylalkohol (28 ml) sowie KOCN (0,247 mol, 20 g) vorgelegt. Zur gerührten Reaktionsmischung wird bei Raumtemperatur schnell (innerhalb 1 min) eine Lösung von 4-Brom-2-fluorbenzylbromid (0,075 mol, 20 g) in DMF (20 ml) mittels Tropftrichter zugegeben. Anschließend wird die erhaltene Suspension unter Rühren auf ca. 110°C erwärmt und bei dieser Temperatur ca. 1,5 h weitergerührt. Danach wird auf Raumtemperatur abgekühlt. Das in der Reaktionsmischung enthaltene Salz wird über eine Fritte abgesaugt. Vom Filtrat wird das Lösungsmittel und überschüssiger Benzylalkohol im Vakuum entfernt. Der leicht gelb gefärbte ölige Rückstand wird zur Kristallisation mit Eis gekühlt. Die anfallende helle kristalline Masse wird mit Wasser gewaschen, filtriert und getrocknet.
Ausbeute: 23,2 g (91,5 % d. Th.)
Fp.: 82 - 84°C
GC/MS: M⁺ = 337, IR in KBr [cm⁻¹]: 3342 (m) N-H, 1689 (s) C=O, 1535 (m) NH-C, 1276 (m) C-O-C

### Beispiel 2

### Synthese von O-Methyl-N-(4-brom-2-fluorbenzyl)carbamat

In einem 1000 ml 2-Hals-Rundkolben mit Rückflußkühler und Trockenrohr werden trockenes und aminfreies DMF (500 ml) und Methanol (50 ml) sowie KOCN (0,62 mol, 50 g) vorgelegt. Zur gerührten Reaktionsmischung wird bei Raumtemperatur schnell eine Lösung von 4-Brom-2-fluorbenzylbromid (0,186 mol, 50 g) in DMF (50 ml) mittels Tropftrichter zugegeben. Anschließend wird die erhaltene Suspension unter Rühren auf ca. 100°C erwärmt und bei dieser Temperatur ca. 1,5 h weitergerührt. Danach wird auf Raumtemperatur abgekühlt. Das in der Reaktionsmischung enthaltene Salz wird über eine Fritte abgesaugt. Vom Filtrat wird das Lösungsmittel und überschüssiges Methanol im Vakuum entfernt. Der ölige Rückstand wird zur Kristallisation gekühlt. Die anfallende helle kristalline Masse wird mit Wasser gewaschen, filtriert und getrocknet.
Ausbeute: 44,3 g (91 % d.Th.)
Fp.: 66 - 67,5°C
GC/MS: M⁺ = 261, IR in KBr [cm⁻¹]: 3338 (m) N-H, 1694 (s) C=O, 1536 (m) NH-C, 1280 (m) C-O-C

Weitere O-Organyl-N-(4-brom-2-fluorbenzyl)carbamate wurden analog Beispiel 1 hergestellt:

| Bsp Nr. | Formel (I) R | Alkohol | Ausbeute [%] | Fp. [°C] | MS | IR (KBr) [cm⁻¹] |
|---|---|---|---|---|---|---|
| 3 | Ethyl | Ethanol | 89 | 39 - 43 | 275 | 3317 (m) N-H, 1686 (s) C = O, 1534 (m) NH-C, 1280 (m) C-O-C |
| 4 | n-Butyl | n-Butanol | 85 | wachsartig | 303 | 3318 (m) N-H, 1695 (s) C = O, 1536 (m) NH-C, 1257 (m) C-O-C |
| 5 | p-Methoxybenzyl | p-Anisalkohol | 89 | 88 - 90 | 367 | 3338 (m) N-H, 1686 (s) C = O, 1536 (m) NH-C, 1278 (m) C-O-C |

## Patentansprüche

1. N-(4-Brom-2-fluorbenzyl)carbamate der allgemeinen Formel (I), 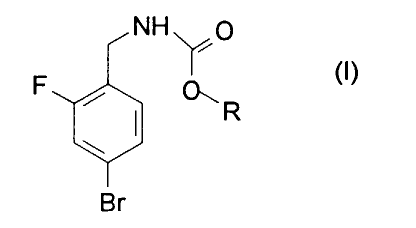 wobei R für (C₁-C₁₂)Alkyl, Benzyl oder Phenyl steht, wobei der Benzyl- und Phenylrest auch durch (C₁-C₃)Alkyl, (C₁-C₃)Alkoxy substituiert sein können.

2. Verbindung nach Anspruch 1, worin in Formel (I) R für (C₁-C₄)Alkyl, Benzyl oder Methoxybenzyl steht.

3. Verbindung nach Anspruch 1, worin Formel (I) für O-Benzyl-N-(4-brom-2-fluorbenzyl)carbamat, O-Methyl-N-(4-brom-2-fluorbenzyl)carbamat, O-Ethyl-N-(4-brom-2-fluorbenzyl)carbamat, O-p-Methoxybenzyl-N-(4-brom-2-fluorbenzyl)carbamat, O-Butyl-N-(4-brom-2-fluorbenzyl)carbamat steht.

4. Verfahren zur Herstellung von Verbindungen der Formel (I), dadurch gekennzeichnet, daß ein 4-Brom-2-fluorbenzylhalogenid mit einem Alkali- oder Ammoniumcyanat in einem dipolar aprotischen Lösungsmittel in Gegenwart des Alkohols ROH umgesetzt wird.

5. Verfahren nach Anspruch 4, dadurch gekennzeichnet, daß als 4-Brom-2-fluorbenzylhalogenid ein Bromid eingesetzt wird.

6. Verfahren nach Anspruch 4 oder 5, dadurch gekennzeichnet, daß als Cyanat Kaliumcyanat eingesetzt wird.

7. Verfahren nach mindestens einem der Ansprüche 4 bis 6, dadurch gekennzeichnet, daß als Lösungsmittel DMF eingesetzt wird.

8. Verfahren nach mindestens einem der Ansprüche 4 bis 7, dadurch gekennzeichnet, daß die Reaktion bei einer Temperatur von 30 bis 150°C, insbesondere 80 bis 140°C, bevorzugt 100 bis 120°C durchgeführt wird.

9. Verfahren nach mindestens einem der Ansprüche 4 bis 8, dadurch gekennzeichnet, daß pro Mol Benzylhalogenid 1,1 bis 3 Mol Cyanat und 1,1 bis 4 Mol Alkohol eingesetzt werden.

## Claims

1. An N-(4-bromo-2-fluorobenzyl)carbamate of the formula (I), 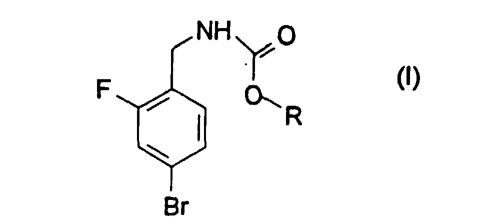 where R is (C₁-C₁₂)alkyl, benzyl or phenyl, where the benzyl and phenyl radicals can also be substituted by (C₁-C₃)alkyl or (C₁-C₃)alkoxy.

2. A compound as claimed in claim 1, wherein in formula (I) R is (C₁-C₄)alkyl, benzyl or methoxybenzyl.

3. A compound as claimed in claim 1, wherein formula (I) is O-benzyl N-(4-bromo-2-fluorobenzyl)carbamate, O-methyl N-(4-bromo-2-fluorobenzyl)carbamate, O-ethyl N-(4-bromo-2-fluorobenzyl)carbamate, O-p-methoxybenzyl N-(4-bromo-2-fluorobenzyl)carbamate or O-butyl N-(4-bromo-2-fluorobenzyl )carbamate.

4. A process for preparing compounds of the formula (I), which comprises reacting a 4-bromo-2-fluorobenzyl halide with an alkali metal cyanate or ammonium cyanate in a dipolar aprotic solvent in the presence of the alcohol ROH.

5. The process as claimed in claim 4, wherein the 4-bromo-2-fluorobenzyl halide used is a bromide.

6. The process as claimed in claim 4 or 5, wherein the cyanate used is potassium cyanate.

7. The process as claimed in at least one of claims 4 to 6, wherein the solvent used is DMF.

8. The process as claimed in at least one of claims 4 to 7, wherein the reaction is carried out at a temperature of 30 to 150°C, in particular 80 to 140°C, preferably 100 to 120°C.

9. The process as claimed in at least one of claims 4 to 8, wherein 1.1 to 3 mol of cyanate and 1.1 to 4 mol of alcohol are used per mole of benzyl halide.

## Revendications

1. Carbamates de N-(4-bromo-2-fluorobenzyle) de formule générale (I) 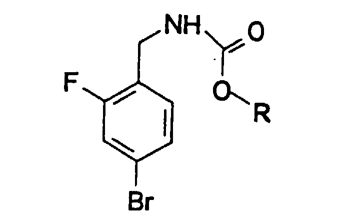 dans laquelle R représente des groupes alkyle en C₁-C₁₂, benzyle ou phényle, le reste benzyle et phényle pouvant être également substitué par des groupes alkyle en C₁-C₃, alkoxy en C₁-C₃.

2. Composé selon la revendication 1, où dans la formule (I), R représente des groupes alkyle en C₁-C₄, benzyle ou méthoxybenzyle.

3. Composé selon la revendication 1, où la formule (I) représente le carbamate d'O-benzyl-N-(4-bromo-2-fluorobenzyle), le carbamate d'O-méthyl-N-(4-bromo-2-fluorobenzyle), le carbamate d'O-éthyl-N-(4-bromo-2-fluorobenzyle), le carbamate d'O-p-méthoxybenzyl-N-(4-bromo-2-fluorobenzyle), le carbamate d'O-butyl-N-(4-bromo-2-fluorobenzyle).

4. Procédé de préparation de composés de formule (I) caractérisé en ce qu'on fait réagir un halogénure de 4-bromo-2-fluorobenzyle avec un cyanate alcalin ou d'ammonium dans un solvant dipolaire aprotique en présente de l'alcool ROH.

5. Procédé selon la revendication 4, caractérisé en ce qu'on emploie un bromure en tant que halogénure de 4-bromo-2-fluorobenzyle.

6. Procédé selon la revendication 4 ou 5, caractérisé en ce qu'on emploie le cyanate de potassium en que cyanate.

7. Procédé selon l'une des revendications 4 à 6, caractérisé en ce qu'on emploie le DMF en tant que solvant.

8. Procédé selon au moins l'une des revendications 4 à 7, caractérisé en ce que la réaction est mise en oeuvre à une température de 30 à 150°C, en particulier de 80 à 140°C, de préférence de 100 à 120°C.

9. Procédé selon au moins l'une des revendications 4 à 8, caractérisé en ce qu'on emploie, par mole d'halogénure de benzyle, de 1,1 à 3 moles de cyanate et de 1,1 à 4 moles d'alcool.
